# EUROPEAN PATENT SPECIFICATION

(11) **EP 1 709 227 B1**
(45) Date of publication and mention of the grant of the patent: **25.02.2009**
(21) Application number: 04815953.7
(22) Date of filing: 30.12.2004
(51) Int. Cl.: D04H 13/00, B32B 5/26, A41D 13/11

(54) **MULTIPLE LAYER NONWOVEN PRODUCTS AND METHODS FOR CREATING COLOR SCHEMES AND FOR PRODUCING SUCH PRODUCTS**
MEHRSCHICHTIGE VLIESSTOFFE UND VERFAHREN ZUR ERZEUGUNG VON FARBZUSAMMENSTELLUNGEN UND ZUR HERSTELLUNG DERARTIGER PRODUKTE
PRODUITS NON TISSES A COUCHES MULTIPLES ET PROCEDES DE CREATION D'AGENCEMENT DE COULEURS ET DE PRODUCTION DESDITS PRODUITS

(30) Priority: 30.12.2003 US 533155 P; 30.12.2003 US 533207 P; 28.12.2004 US 27701
(43) Date of publication of application: 11.10.2006
(73) Proprietor: KB Aviation, Inc., Aledo TX 76008 (US)
(72) Inventor: BRUNSON, Kevin, K., Aledo, Texas 76008 (US); CALDWELL, Darell, S., Glen Rose, Texas 76043 (US)
(74) Representative: Hedley, Nicholas James Matthew
(86) International application number: PCT/US2004/043964
(87) International publication number: WO 2005/066406

(56) References cited:
- WO-A-02/00426
- WO-A-95/15410
- WO-A-96/10380
- WO-A-98/06562
- WO-A-99/46119
- DE-A1- 4 106 295
- US-A- 4 211 593
- US-A- 6 055 982
- US-A1- 2003 092 341
- PATENT ABSTRACTS OF JAPAN vol. 015, no. 163 (C-0826), 24 April 1991 (1991-04-24) & JP 03 033217 A (KURARAY CO LTD), 13 February 1991 (1991-02-13)

## Description

### Technical Field

The present invention is related to colored nonwoven materials and more particularly to products formed, at least in part, from colored nonwoven materials and methods for creating color schemes in such products.

### Background Art

Nonwoven materials or fabrics have been used for many years to produce a wide variety of products. Examples of such products include, but are not limited to, agricultural products such as sacks, crop covers, ground cover and weed barriers, building materials (insulation, house wraps and roofing materials), healthcare related products (caps, gowns, facial protection masks, drapes and shoe covers), respirator masks, apparel, biological protection suits, personal care products, wipes for polishing, cleaning and disinfecting, diapers, floor covering, packaging and automobile headliners and upholstery.

Nonwoven materials may be flat sheets or web type structures formed by bonding or entangling individual fibers and filaments using appropriate mechanical, thermal and/or chemical processes. Nonwoven materials may also be formed by perforating relatively thin layers of plastic film. Nonwoven materials may be combined with thin plastic films without apertures or perforations and/or nonwoven materials. Various techniques and procedures may be used to produce porous nonwoven materials and fluid resistant nonwoven materials. Nonwoven materials do not require weaving or knitting and do not require converting individual fibers or filaments into yarn.

Nonwoven materials may have a limited, single use life or may have a long, multiple use life. Nonwoven materials may be designed for specific features or functions such as absorbency, fluid resistance, resilience, softness, strength, flame resistance, cushioning, chemical resistance, filtration and bacteria or anti-microbial barrier and combinations thereof. However, these features and performance capabilities can generally only be determined by appropriate testing and inspection. Visual appearance of nonwoven materials is generally unable to identify specific features and performance characteristics.

Products may be created by combining appropriate nonwoven materials to meet specific applications while at the same time optimizing the useful life and cost of the resulting products. Nonwoven materials may have substantially the same strength, texture and appearance as woven materials. Nonwoven materials and nonwoven products may be used to provide a wide range of products.

A wide variety of techniques and procedures may be used to produce nonwoven materials and fabrics. Textile based technologies such as garneting, carding, and needle punching may be used to form fibers and filaments into selectively oriented webs. Fabrics produced by these systems may sometimes be referred to as drylaid nonwovens. Textile based nonwoven materials may be manufactured with machinery designed to manipulate textile fibers in a dry state.

Paper based technologies such as drylaid and wetlaid systems designed to accommodate short synthetic fibers and filaments similar to wood pulp fibers may be used to produce nonwoven materials. Fabrics produced by these systems may sometimes be referred to as drylaid and wetlaid nonwovens. Paper based nonwoven materials may be manufactured with machinery designed to manipulate short fibers suspended in fluid. Such paper based nonwovens may sometimes be referred to as tissues.

Extrusion based technologies such as spunbond, meltblown, and porous and nonporous film systems may also be used to produce nonwoven materials. Fabrics produced by these systems may sometimes be referred to as spunbonded, meltblown, and textured or apertured and non-apertured film nonwovens. The term "polymer laid nonwovens" may be used to describe all extrusion based nonwovens. Extrusion based nonwovens may be manufactured with machinery associated with polymer extrusion. Fiber structures may be simultaneously formed and manipulated by many polymer laid systems.

Hybrid technologies such as hydroentangling which combine one or more techniques associated with textile based technologies, paper based technologies and extrusion based technologies may also be used to produce nonwoven materials. Nonwoven materials may be formed with a single layer or single web type structure. Nonwoven materials may also be formed with two or more sublayers or web type structures.

Various printing techniques and dyeing techniques have previously been used to produce colored nonwoven materials. Conventional color printing and dyeing techniques associated with nonwoven materials and fabrics generally do not create an appearance of depth or thickness.

Some manufacturers of nonwoven materials have used various shades of blue and green coloring to indicate basis weight of associated products. Manufacturers of colored nonwoven materials often take extra steps to produce webs of nonwoven material as uniform in color as possible. Frequently, pigments are carefully added to each web or layer of nonwoven material to produce a balanced, uniform color. Wrapping materials and shipping materials associated with products formed from nonwoven materials have previously been color coded to indicate performance characteristics such as sterilization, storage and handling process for products which will be used in a sterile or surgical-type environment.

One fabric currently on the market is a spunbond meltblown spunbond (hereinafter referred to as "SMS") polypropylene three layer composite having a blue meltblown layer sandwiched between blue and white spunbond layers. The colors of the two blue layers appear to be the same color. It is believed that products constructed from this material have the blue layer on the outside of the products and the white layer on the inside. The white inner layer is not visible through the blue outer layer, although the blue middle layer is somewhat visible through the white inner layer. There are also instances where SMS has been made where each of the three layers are substantially the same color but slightly different shades thereof because of manufacturing imperfections and/or differences in associated manufacturing processes.

Layers of nonwoven materials with different colors, particularly two layers of nonwoven material, have often designed such that the color of one layer is not normally visible relative to the color of another layer. Significant manufacturing and quality control procedures have been used to prevent the color of one nonwoven layer from being visible through a nonwoven layer with a different color. Density, basis weight, thickness and/or loft of each nonwoven layer are often selected to occlude or prevent cooperation between the color of one layer and the color of an adjacent layer. Fiber diameter may be another factor that determines translucent or opaque characteristics of a nonwoven layer or sublayer.

Two or more layers or two sublayers of nonwoven material have often been formed with substantially the same color to avoid highlighting or showing void spaces or "thin spaces" associated with many types of nonwoven materials. Forming two or more layers or sublayers of nonwoven material with substantially the same color has often been used to provide the general appearance of uniformity and durability associated with more conventional woven materials. The same color of one layer or sublayer has been used to cooperate with the same color of an adjacent layer or sublayer to provide the desired appearance of uniformity. Again, significant manufacturing and quality control procedures have been used to prevent differences or variations between the color of one or more nonwoven layers or sublayers which are visible through one or more adjacent nonwoven layers or sublayers with substantially the same color.

One class of products of interest is medical and facial protection products. Medical and dental facial protection products are frequently used on a routine basis for many medical, dental and healthcare related activities. Surgical face masks are representative of such products. The dramatic increase of infectious diseases, such as SARS, AIDS, Avian Flu and tuberculosis has resulted in substantially increased use of face masks by both healthcare professionals (doctors, nurses, dentists) and other people working in a healthcare environment. Also, face masks are frequently worn by members of the general public as a result of concern about SARS, Avian Flu, multiple drug resistant tuberculosis (MDR-TB) and other infectious diseases.

Medical and dental facial protection products are often formed from nonwoven materials designed to provide specific features and performance capabilities such as fluid resistance, softness, filtration and bacteria or anti-microbial barriers. However, these features and performance capabilities can generally only be determined by appropriate testing and inspection. Visual appearance of a nonwoven material is generally unable to identify specific features and performance characteristics. Manufacturers of nonwoven materials for medical and dental facial protection products also often take extra steps to produce webs of nonwoven material as uniform in color as possible.

Various types of nonwoven materials have frequently been combined with each other to meet the increasing need for facial protection in the medical, dental and healthcare related activities. Also, various printing techniques and dyeing techniques have previously been used to produce colored nonwoven materials used to form medical and dental facial protection products. Some face masks have previously been produced with one color or color pattern visible on one surface or side of the face mask and a different color or color pattern visible on an opposite surface or side of the face mask.

Various types of nonwoven products have been formed with a white inner layer of nonwoven material to indicate an interior surface or inner lining. For example, some face masks have been formed with a white interior surface to indicate where the face masks are to contract a wearer's face and a generally orange outer layer to indicate fluid resistant characteristics of the face mask. However, the white inner layer and the generally orange outer layer did not cooperate with each other to produce a color scheme. In addition, a filtration layer is situated between the white inner layer and the orange outer layer. Also, coats, jackets and other garments have been formed with a white layer of nonwoven material to form an interior surface or inner lining for the associated garment. The outer layer or layers of such garments are often formed from nonwoven material having various colors such as yellow or blue.

However, inner white layer and the outer colored layer or layers did not generally cooperate with each other to produce a color scheme. Frequently, pigments are carefully added to each outer web or layer of nonwoven material to produce a balanced, uniform color.

WO 96/10380 discloses a laminate material having an outer and inner layer of material. The outer layer includes transparent areas so that the lower layer is visible.

WO 02/004262 discloses a nonwoven fabric with variable fibre density and selected patterns where the fabric is further laminated to a colored backing material.

US 4211593 discloses a product formed by needling a stack of differently-colored nonwoven fleeces such that the fibres of a lower layer are brought to the surface to form a pattern.

DE 4106295 describes a fibre web attached to a carrier web by needle punching. The fibre web can have cuts or incisions and the webs can be formed of different colors to give a patterned or sculptured non-woven fabric.

US 2003/0092341 describes a camouflage material that is printed or dyed to a certain pattern.

### Disclosure of Invention

The present invention is as defined in the accompanying claims.

In accordance with teachings of the present invention, nonwoven products may be formed with one or more layers of nonwoven material having a selected color scheme. The nonwoven material may be formed from fibers, films or filaments, including combinations thereof. The color scheme may be formed by differentially pigmenting fibers or filaments in one or more of the layers of nonwoven material. The color scheme may also be formed by differentially pigmenting a plastic film. Differences in color or color schemes may be used to provide a visual appearance of depth or thickness for an associated nonwoven product. Color schemes formed in accordance with teachings of the present invention may provide a visual indication of desired functions, features or performance characteristics of an associated product. For example, medical clothing or garments appropriate for use in potentially contaminated environments may have a unique color scheme to indicate that gowns, booties, shoe covers, masks, gloves, head covers and/or coveralls meet applicable anti-microbial or fluid resistant requirements for the specific environment. In a similar manner color schemes may be established to indicate that coveralls, shoe covers, masks, gloves and other apparel meet the specific requirements for use in clean rooms. Color schemes may be selected to indicate products which meet various barrier requirements with respect to particulate matter, fluids, chemical hazards, bacteria and/or viruses. Color schemes and variations in color schemes may be used to indicate the size of a nonwoven product.

One aspect of the present invention includes differentially pigmenting the fibers or filaments in each layer or sublayer of nonwoven material such that the same type of nonwoven material may be used for each layer or sublayer of the resulting product and still provide a desired color scheme. Fibers, filaments and/or films may be differentially pigmented by varying any of a number of factors, including pigment color and concentration. The overall color or color scheme of a layer may also be varied by changing fiber or filament diameter, filament or fiber density, coverage or spacing. More than one characteristic may be varied between layers or sublayers. Additionally, some embodiments may have multiple colors or shades of color within the same layer or sublayer.

Embodiments of the present invention may allow reduction of the total amount of pigments which must be added to fibers or filaments used to form nonwoven materials while providing desired color schemes. In selected embodiments, the amount of pigments which must be added to nonwoven fibers as a percentage of the fiber denier per basis weight may be reduced. Often, less than five percent (5%) by weight of pigments will be added to a layer or sublayer of nonwoven material. The concentration of pigments may vary with respect to fibers or filaments of each layer.

For some applications, nonwoven products may be formed with multiple layers of colored nonwoven materials which cooperate with each other to produce a color scheme which provides visual identification of various functions, features, characteristics, size or performance capabilities of associated nonwoven products. Examples include, but are not limited to, each type of use associated with wipes formed from nonwoven materials indicated by respective color schemes. Each level of protection provided by chemical or biohazard suits may be indicated by respective color schemes formed in associated nonwoven materials in accordance with teachings of the present invention. Alternatively, nonwoven products may be formed with sublayers or webs which have different concentrations of pigments to produce color schemes which cooperate with each other to produce a color scheme indicating associated functions, features, characteristics or performance capabilities of the associated nonwoven product. The sublayers or webs may be formed with the same technology (spunbonded, meltblown, wet laid, dry laid, hydroentangled or extruded) or may be formed from different technologies, one sublayer spunbonded, one sublayer meltblown. Within each layer or sublayer, the concentration of pigments may vary substantially.

One aspect of the present invention includes forming nonwoven products with a layer of meltblown material disposed between a first layer of spunbonded material and a second layer of spunbonded material.

Another aspect of the present invention includes forming nonwoven material from bicomponent fibers or filaments having a core disposed within a hollow sheath or tubing.

For many bicomponent materials approximately sixty to ninety percent of the basis weight may be provided by the core and approximately ten to forty percent of the basis weight may be provided by the sheath. The present invention includes adding color pigments primarily to the sheath and not to the core which reduces total quantity of pigments required to produce a desired color or color scheme in nonwoven materials formed from bicomponent fibers or filaments.

Technical benefits of the present invention include eliminating or substantially reducing potential damage to meltblown nonwoven materials by substantially reducing the amount or quantity of pigments which must be added to the meltblown fibers or filaments. Another technical benefit of the present invention includes providing nonwoven products with specific color schemes which provide a visual indication of functions, features, characteristics or performance capabilities without requiring inspection and testing of the nonwoven product.

A further aspect of the present invention includes varying the color concentration of pigments added to fibers or filaments used to form a layer or sublayer of nonwoven material. Products with various color schemes may then be fabricated by using multiple layers of nonwoven material with each layer having a different color scheme. Color or color scheme may also be varied by varying the diameter of fibers or filaments, the density of fibers or filaments, coverage and spacing, inter alia.

Technical benefits of the present invention include forming color coded garments and accessories which may be worn or used by personnel working in a specific environment, such as a medical or clean room environment. For example, colored nonwoven materials may be used to develop a first color coding system for products appropriate for use in surgical suites and a second color coding system for products appropriate for use in highly infectious isolation areas, such as Avian Flu, tuberculosis and SARS wards. Materials of the present invention may also be used to form medical products such as bandages and wraps. In another example, colored nonwoven materials may be used to code bandages having different therapeutic compositions, such as antiseptics or antibiotics. Variations in color schemes for a nonwoven product such as personnel protection equipment (PPE) and/or biohazards suits may be used to indicate sizes. Such PPE and/or biohazard suits may be more quickly donned during an emergency condition.

Nonwoven materials incorporating teachings of the present invention may be satisfactorily used to form products such as respirators for medical and other uses, including industrial uses disposable diapers, personal care products such as sanitary napkins and tampons, sterilization wraps, masks, gowns, caps and drapes associated with the healthcare industry, other disposable garments, household and personal wipes, apparel interlining, carpeting and upholstery fabrics, padding and backing, wall coverings, agricultural coverings and seed strips, automotive headliners and upholstery, filters, envelopes, tags, labels, packaging, insulation, house wrap and roofing materials, geotextiles, car or equipment covers, shade covers, other outdoor materials, bright colored safety garments, bandages and wraps, and other medical devices.

In accordance with one form of the present invention, medical and dental facial protection products may be formed with at least two layers or sublayers of nonwoven material and at least one of the layers or sublayers of nonwoven material having a different color or color scheme. Alternatively, a layer of nonwoven material with a color scheme incorporating teachings of the present invention may be combined with one or more layers of woven material to form a facial protection product. The color scheme may be formed by differentially pigmenting fibers or filaments in one or more layers of nonwoven material. A single layer or sublayer of nonwoven material may have different colors.

Differences in color, including differences in shading and/or tint of the same color, and specific color schemes may be used to provide a visual indication of desired functions, features, characteristics or performance capabilities of an associated medical or dental facial protection product. For example, color schemes formed in accordance with teachings of the present invention may be used to indicate anti-microbial characteristics or fluid resistant characteristics of associated medical or dental facial protection products.

Anti-microbial and fluid resistance requirements may change substantially depending upon the specific environment in which facial protection products will be used. The present invention allows developing a color scheme to indicate facial protection products which satisfy specific requirements for many different environments and levels of risk associated with medical, dental and healthcare related activities. Color schemes formed in accordance with teachings of the present invention may indicate that facial protection products meet specific barrier requirements with respect to particulate matter from fluids, bacteria and/or viruses.

One aspect of the present invention includes forming medical and dental facial protection products from multiple layers or sublayers of nonwoven material. For example, a face mask may be formed in accordance with teachings of the present invention with a layer of meltblown material disposed between a first layer of spunbonded material and a second layer of spunbonded material. Alternatively, a layer of nonwoven material may be formed with two or more sublayers, each having a different color or color scheme. A color scheme formed on a face mask in accordance with teachings of the present invention may be a one layer or sublayer of nonwoven material having a color scheme corresponding with robin's egg blue. Another layer or sublayer of nonwoven material may have a color scheme based on wisteria violet. A third layer or sublayer of nonwoven material may have a color scheme based on seafoam green.

A further aspect of the present invention may include forming one or more layers of nonwoven material with colors selected to substantially reduce or eliminate glare associated with typical face masks and visors. One or more layers of such nonwoven material may be placed on a face mask adjacent to the wearer's eyes to minimize or prevent glare.

In accordance with one form of this invention, there is provided a nonwoven product including a first layer and a second layer. The first layer may be disposed adjacent to the second layer. The second layer forms an outer visible surface of the product. At least a portion of the first layer has a first color scheme. At least a portion of the second layer has a second color scheme.
The first color scheme is different from the second color scheme. The second layer is constructed so that at least a portion of the first color scheme is visible under normal lighting conditions when viewed through at least a portion of the second layer. The first color scheme cooperates with the second color scheme to produce a third color scheme which is different from the first and second color schemes.

In accordance with another form of this invention, there is provided a nonwoven product including at least first and second layers or sublayers made from nonwoven material. The first and second layers or sublayers are differentially pigmented wherein the first layer is a first color and the second layer or sublayer is a second color. The second layer or sublayer may form an outer layer of the product. The first and second layers or sublayers cooperate with one another to create a visual image of a combination of the first and second colors on the second layer or sublayer. For some applications the basis weight (grams per square meter) of the outer layer or sublayer may be less than the basis weight of adjacent layers or sublayers to allow cooperation between the respective colors. The basis weight of each layer or sublayer may be varied from the outer layer or sublayer having the lightest or smallest basis weight and each adjacent layer or sublayer having a heavier or larger basis weight to produce a desired color scheme.

In accordance with another form of this invention, there is provided a nonwoven product including an inner layer and an adjacent outer layer. The inner layer is a first color and the outer layer is a second color. The first color is different from the second color. At least a portion of the outer layer is translucent. The translucence or opacity of the outer layer is such that the color of the inner layer is visible through at least a portion of the outer layer under normal working conditions to produce a desired color scheme when viewed from the outer layer.

In accordance with another form of this invention, there is provided a nonwoven product including an inner layer and an adjacent outer layer. The inner layer is of a different color from the color of the outer layer. The outer layer is constructed so that the color of the inner layer is visibly discernable through at least a portion of the outer layer. The first color and the second color cooperate with each other to produce a color scheme when viewed from the outer layer.

In accordance with another form of this invention, there is provided a method for forming a nonwoven product. A first color is applied to a first material. The first material is deposited on a substrate, thereby forming a first layer. A second color is applied to a second material. The second material is deposited on the first layer, thereby forming a second layer. The first and second layers form a fabric. Constructing the product so that the second layer is the outer layer. The translucence of the second layer being such that the first color is visible through at least portions of the second layer.

In accordance with another form of this invention, there is provided a method for forming a nonwoven product. A first material is deposited on a substrate, thereby forming a first layer. At least a first color is applied to the first material. A second material is deposited on the first layer, thereby forming a second layer. A second color is applied to the second material. The first and second layers form a fabric. Constructing the product so that the second layer is the outer layer. The translucence of the second layer is such that the first color is visible through at least portions of the second layer.

In accordance with another form of this invention, there is provided a nonwoven product including a first layer made of nonwoven material and a second layer made of nonwoven material. The first layer and the second layer are adjacent to one another. The first layer is a first color and the second layer is a second color. At least portions of the second layer are translucent so that the first color is visible through the second layer.

In accordance with another form of this invention, there is provided a nonwoven product including at least first, second and third layers, each made of nonwoven material. The second layer is sandwiched between the first and third layers. The first layer is a first color, the second layer is a second color and the third layer is a third color. The first, second and third colors are different colors. At least a portion of the first layer is translucent and at least a portion of the second layer is translucent so that the second and third colors are visible through the first layer. For some applications, colors associated with four or more layers of nonwoven material may be visible through the first layer.

In accordance with another form of this invention, there is provided a nonwoven product including first, second and third layers made of nonwoven materials. The second layer is sandwiched between the first and third layers. The first layer is a first color, the second layer is a second color and the third layer is a third color. The first and third colors are substantially the same. The second color is different from the first and third colors. At least a portion of the first layer is translucent and at least a portion of the third layer is translucent so that the second color is visible through the first layer and through the third layer.

One aspect of the present invention may be forming facial protection masks, medical and dental face masks, medical respirators and industrial respirators from nonwoven materials and nonwoven composites with color schemes in accordance with teachings of the present invention.

Another aspect of the present invention may be forming medical and dental products including, but not limited to, caps, gowns, head covers, shoe covers, surgical drapes, sterilization wraps, ice packs, bandages, wound dressings, medical uniforms and protective garments worn in surgery (scrubs) with color schemes in accordance with teachings of the present invention.

Another aspect of the present invention may be forming non-medical and non-dental products and non-respirator products including, but are not limited to, agricultural products, industrial products, home building products, outdoor products, personal protection equipment (PPE) and personal care products with nonwoven materials having color schemes in accordance with teachings of the present invention. Examples of such agricultural products may include, but are not limited to, crop covers, sacks, ground covers, weed barriers and seed strips. Examples of such industrial products may include, but are not limited to, equipment covers, filters, uniforms, wrapping materials, utility straps, vehicle covers, labels, tags, packaging, envelops, vehicle headliners, wipes for polishing, cleaning and/or disinfecting and padding. Examples of such home building products may include, but are not limited to, insulation, house wraps, roofing materials, wall coverings and floor pads. Examples of personal protection equipment may include uniforms, hoods, head covers, gowns, shoe covers, vests, chemical suits, biological protection suits, firefighting suits, survival suits and biohazard suits. Examples of outdoor products may include, but are not limited to, shade coverings, awnings, camouflage materials, rain gear lawn and patio furniture, tents, disposable towels, blankets and apparel. Examples of personal care products may include, but are not limited to, disposable diapers, undergarments, sanitary napkins, tampons, feminine hygiene products, and body wipes. Examples of other non-medical and non-dental products and non-respirator products may include, but are not limited to, apparel, upholstery, backing materials, interlinings and veterinary care products such as bedding, animal covers, pet garments, stall liners, animal wraps and stabilizers.

### Brief Description Of The Drawings

FIGURE 1 is a schematic drawing in elevation showing a medical, dental or industrial respirator incorporating teachings of the present invention;
FIGURE 2 is a schematic drawing in elevation showing a side view of the respirator of FIGURE 1;
FIGURE 3 is a schematic drawing in section taken along lines 3-3 of FIGURE 2;
FIGURE 4 is a schematic drawing in elevation with portions broken away showing a shoe cover formed from nonwoven materials incorporating teachings of the present invention;
FIGURE 5 is a schematic drawing in elevation showing a hair cover formed from nonwoven materials incorporating teachings of the present invention;
FIGURE 6 is a schematic drawing showing a surgical coat formed from nonwoven materials incorporating teachings of the present invention;
FIGURE 7 is a schematic drawing with portions broken away showing one example of multiple layers of nonwoven material having different color schemes in accordance with teachings of the present invention;
FIGURE 8 is a schematic drawing with portions broken away showing a bicomponent fiber formed in accordance with teachings of the present invention;
FIGURE 9 is a schematic drawing in elevation showing a face mask formed in part from nonwoven materials incorporating the teachings of the present invention;
FIGURE 10 is a schematic drawing in elevation showing another example of a face mask formed in part from nonwoven materials incorporating the teachings of the present invention;
FIGURE 11 is a schematic drawing in elevation showing a visor and face mask formed in part from nonwoven materials incorporating the teachings of the present invention;
FIGURE 12 is a pictorial view of a two layer fabric formed in accordance with the teachings of the present invention;
FIGURE 13 is an elevational view of the fabric of FIGURE 12;
FIGURE 14 is a sectional view of the fabric of FIGURE 13 taken through section lines 14-14 illustrating the visual discernability of the colors from the fabric;
FIGURE 15 is a pictorial view of a three layer fabric formed in accordance with the teachings of the present invention;
FIGURE 16 is a sectional view of the fabric of FIGURE 15 and illustrating the color transmission of the fabric;
FIGURE 17 is an isometric view of a trilobal filament with different colors on each lobe in accordance with the teachings of the present invention;
FIGURE 18 is a schematic drawing showing a hospital gown formed from nonwoven materials incorporating the teachings of the present invention;
FIGURE 19 is a schematic drawing showing a biological hazard suit formed from nonwoven materials incorporating the teachings of the present invention;
FIGURE 20 is a schematic drawing showing a diaper worn by an infant formed from nonwoven materials incorporating the teachings of the present invention;
FIGURE 21 is schematic drawing showing a surgical drape formed from nonwoven materials incorporating the teachings of the present invention;
FIGURE 22 is a schematic drawing showing a feminine hygiene product formed from nonwoven materials incorporating the teachings of the present invention;
FIGURE 23 is a schematic drawing showing a wipe formed from nonwoven materials incorporating the teachings of the present invention;
FIGURE 24 is a schematic drawing showing a healthcare worker wearing a head cover, a surgical mask, a gown and shoe covers all of which are made from nonwoven materials incorporating the teachings of the present invention;
FIGURE 25 is a block diagram showing a method for producing a nonwoven fabric incorporating teachings of the present invention; and
FIGURE 26 is a pictorial view of an apparatus, in a simplified form, which may be used for carrying out a method incorporating teachings of the present invention.

### Best Modes for Carrying Out the Invention

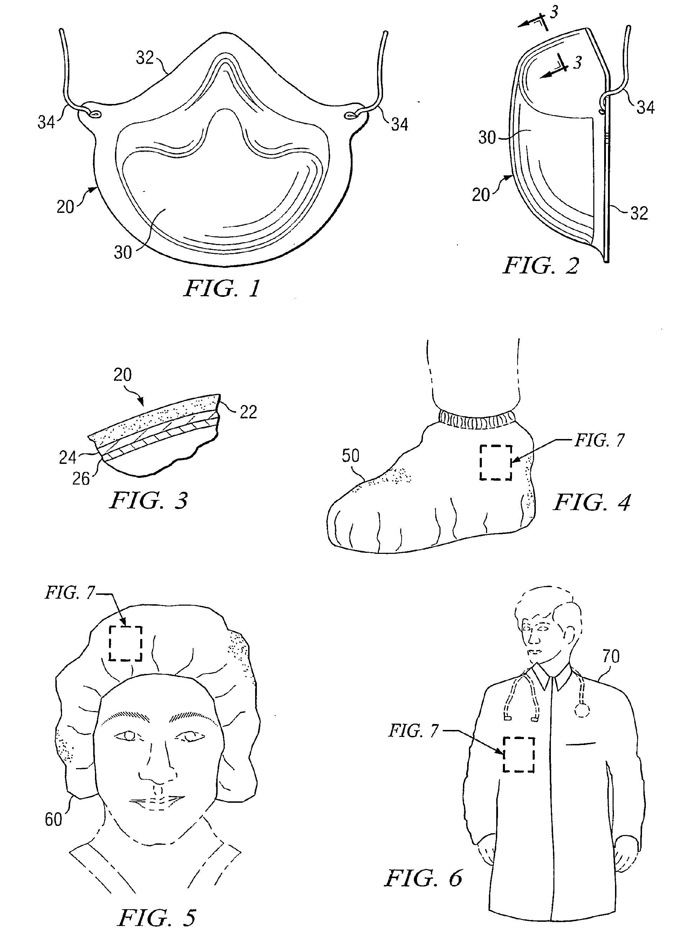

Preferred embodiments of the invention and its advantages are best understood by reference to FIGURES 1-26 wherein like numbers refer to same and like parts.

The terms "nonwoven product" and "nonwoven products" as used in this application include, but are not limited to, respirators including medical respirators and industrial respirators, disposable diapers, sanitary napkins and tampons, sterilization wraps, medical and dental facial protection products including face masks and any products formed at least in part from nonwoven materials for use in any medical, healthcare or dental applications, gowns, caps and drapes associated with the healthcare industry, other disposable garments, household and personal wipes, apparel interlining, floor covering and upholstery fabrics, padding and backing, wall coverings, agricultural coverings and seed strips, automotive headliners and upholstery, filters, envelopes, tags, labels, insulation, house wraps, roofing materials, geotextiles, car or equipment covers, shade covers, other outdoor materials, bandages and wraps, and other medical devices formed at least in part from nonwoven materials, and nonwoven composites. The term "nonwoven composites" may be used to describe nonwoven materials combined with thin plastic films with or without perforations or apertures and/or woven materials.

The term "pigment" as used in this application shall mean any finely powdered natural or synthetic color material which may be satisfactorily used to add color to fibers, filaments and plastic films used to form nonwoven materials. Pigments are often mixed with oil, water or other suitable liquids to form paint, ink or dye. One aspect of the present invention includes varying the concentration of pigments directly added to fibers, filaments and plastic films during the process of forming respective layers or sublayers of nonwoven materials and/or fabrics.

The term "spunbonded", as used in this application, includes nonwoven materials and fabrics formed by extrusion of polymer based filaments or fibers which may then be laid down in the form of a web and subsequently bonded.

The term "meltblown", as used in this application, includes nonwoven materials and fabrics formed by melt extruding a polymer through a die into a high velocity stream of hot air which converts the polymer into fine, relatively short fibers or filaments. The fibers or filaments may be collected on a sheet on a moving screen and later bonded with each other or other materials.

The term "nonwoven materials" as used in this application also includes nonwoven fabrics. Nonwoven materials and fabrics may be flat sheets or web type structures formed by bonding or entangling separate fibers or filaments using appropriate mechanical, thermal and/or chemical processes. Nonwoven materials and fabrics may also be formed, at least in part by, plastic films.

Various techniques and procedures may be used to produce porous nonwoven materials and fluid resistant nonwoven materials. Some nonwoven materials are sometimes referred to as "spunbonded" or "meltblown" as an indication of the associated techniques and procedures used to form the respective nonwoven material. Other nonwoven materials may sometimes be formed by extruding relatively thin plastic films. Some plastic films may be perforated. Other plastic films may have no holes or apertures. Nonwoven materials do not require weaving or knitting and do not require converting individual fibers or filaments into yarn. Other processes may be used to produce nonwoven materials. For example, nonwoven materials and fabrics may be wet laid, dry laid or hydroentangled.

Accordingly, the term "fibers or filaments" may include mixtures of primarily fibers, primarily filaments or combinations thereof as formed or used in the selected technique for producing nonwoven materials.

The term "color scheme" is used in this application to include any combination or arrangement of colors, shades of colors within one or more layers or sublayers of nonwoven material or a nonwoven composite to produce a desired random visual appearance. The color schemes create a generally random appearance with respect to changes in different colors or changes in shading or tint of the same color within a layer or sublayer of nonwoven material or a nonwoven composite. Such apparently random changes in color shading or tint may be desirable to produce an appearance of thickness or depth. A matrix of different colors or a matrix of different tints or shadings of the same color may be used to produce a desired color scheme in multiple layers or sublayers of nonwoven material or a nonwoven composite. Also, a color scheme incorporating teachings of the present invention may include one layer or sublayer having a generally uniform color in combination with other layers or sublayers having variations in color to provide a desired visual indication of performance characteristics of the resulting nonwoven product. Substantial variations in shading or tint may also be used in one or more layers or sublayers.

FIGURES 1 through 24 show various examples of nonwoven products, nonwoven materials and fibers or filaments formed in accordance with teachings of the present invention. Respirator 20, as shown in FIGURES 1, 2 and 3, may generally be described as a medical respirator, a dental respirator or as an industrial respirator depending in part upon performance characteristics of nonwoven materials used to form respirator 20. Multiple layers of material 22, 24 and 26 may be molded with each other to produce relatively rigid central portion 30 and relatively thin, flat peripheral portion 32. For some applications, central portion 30 may have a generally "cup-shaped" configuration to accommodate the face of a wearer and provide an air exchange chamber. Peripheral portion 32 may be relatively thin and flat to form a seal with the face of a wearer. Various types of ties, bands and/or strings 34 may be attached with peripheral area 32 for use in securing respirator 32 with the face of a wearer (not expressly shown). A color scheme formed in accordance with teachings of the present invention is preferably visible from the exterior of respirator 20 to provide a visual indication of associated features and performance characteristics appropriate for use in various medical, dental and industrial environments.

Layers 22, 24 and 26, as shown in FIGURE 3, may be formed from various types of nonwoven materials which cooperate with each other to form a color scheme incorporating teachings of the present invention. For some applications, only layer 22 may be formed from nonwoven material having a color scheme incorporating teachings of the present invention. Alternatively, one or more layers 22, 24 or 26 may be formed from sublayers or webs of nonwoven material which cooperate with each other to form a color scheme. For example, layer 22 may be formed from two or more sublayers which produce a desired color scheme.

Extrusion based technologies such as spunbonded, meltblown, porous and nonporous film systems may be used to produce nonwoven materials.

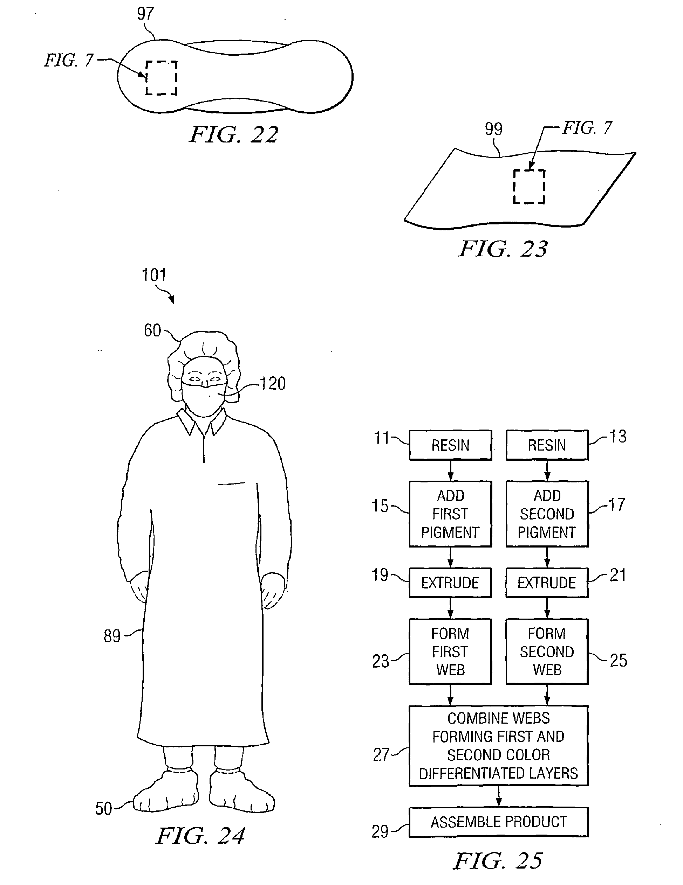

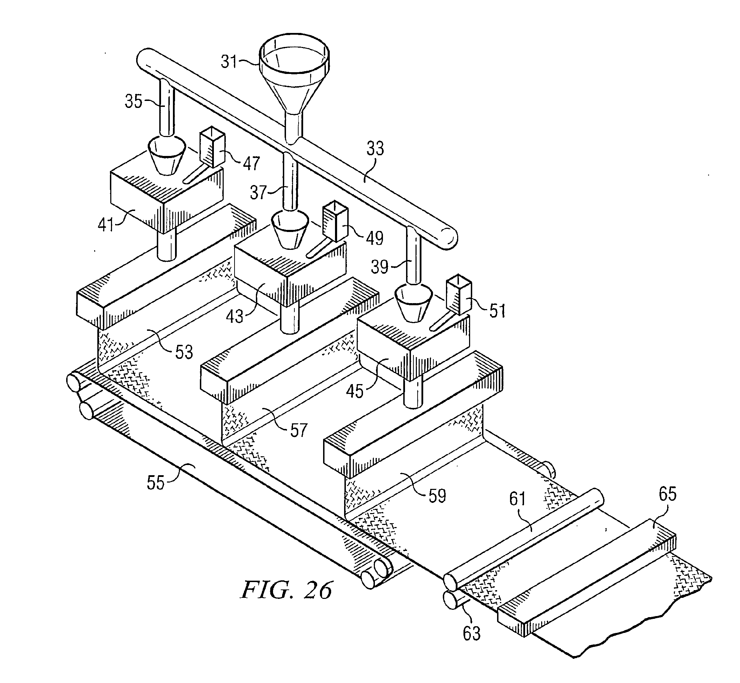

In reference to FIGURES 25 and 26, products and fabric constructed from multi-colored layers incorporating the teachings of the present invention may be made using method and apparatus discussed below.

Referring now more particularly to FIGURE 25, which is a block diagram illustrating a method for producing two layer fabric. Blocks 11 and 13 demonstrate that the method starts with a resin, such as polypropylene. A first pigment is added to one of the resins, as illustrated by block 15, and a second pigment is added to the other resin as illustrated by block 17. The first and second pigments may be different colors or different shades of the same color. The resin colored with the first pigment is then extruded as illustrated by block 17 into multiple filaments as illustrated by block 19. As illustrated by block 21, the resin containing the second pigment is also extruded into a plurality of filaments. The extruded filaments from block 19 form a first web as illustrated by block 23 and the extruded filaments from block 21 form a second web as illustrated by block 25. As illustrated by block 27, the webs are combined, thereby forming first and second differentiated layers. As illustrated by block 29, the two layered fabric may then be used to assemble a nonwoven product, such as the products referred to above.

FIGURE 26 illustrates an apparatus for producing three layers having two layers such as of spunbond polypropylene with a layer of meltblown polypropylene sandwiched therebetween, also known as SMS. Hopper 31 is provided for receiving pigmented polypropylene chips. Hopper 31 is connected to manifold 33 having outlets 35, 37 and 39 disbursing the polypropylene chips into extruders 41, 43 and 45. Extruders 41 and 45 may be spunbond extruders and extruder 43 may be a meltblown extruder. Pigment containers 47, 49 and 51 may be connected to extruders 41, 43 and 45 for adding differentially colored pigment to the extruders. For some embodiments the colors in containers 47 and 51 may be the same or substantially the same. First web 53 of spunbond polypropylene may be laid onto a moving substrate or belt 55 forming a first layer or web of nonwoven material of a first color. Extruder 43 forms a layer of meltblown polypropylene 57 which may be deposited on top of layer 53 and a layer of spunbond polypropylene 59 may be deposited on top of meltblown layer 57. Rollers 61 and 63 calendar the three layers into a multi-colored SMS fabric 65. It may be preferred that the nonwoven materials, in particularly the nonwoven material forming the outer layer of the assembled product, have sufficient opacity or translucence such that a desired color scheme may be visible when the assembled product is viewed from the outer layer. The opacity or translucence of each layer or sublayer may be determined by varying one or more of the following characteristics of the nonwoven materials: fiber or filament diameter, fiber or filament density and/or basis weight of each layer, fiber or filament shape, pigment concentration, web coverage, fiber dispersion and/or color selection.

In a specific embodiment, fibers or filaments associated with spunbonded and meltblown nonwoven materials are often formed using an extrusion head or an extrusion beam. A hopper is typically associated with each extrusion head or extrusion beam for use in adding pigments to the polymeric material during the process of forming respective fibers and filaments. For example, a first hopper and extrusion head or extrusion beam may cooperate with each other to add approximately five to six percent by basis weight of pigments to the associated fibers or filaments. A second hopper and associated extrusion head or extrusion beam may be used to add three or four percent of pigments by basis weight to the associated fibers or filaments. A third hopper and extrusion head may be used to add one or two percent by basis weight of pigments to the associated fibers or filaments. The extrusion heads may be used to form separate layers of nonwoven material or they may be used to form sublayers of nonwoven material. Alternatively, the extrusion heads may cooperate with each other to form sublayers or webs of respective spunbonded meltblown and spunbonded material. The size of the fibers used to form each layer and the concentration of pigments are selected to provide a visual indication of the performance characteristics of the product, such as a facial protection product, formed from the nonwoven material.

However, in other applications it may be possible to construct a series of valves (not expressly shown) that allow connection of multiple hoppers to a single extrusion head to produce fibers or filaments with discontinuous pigmentation. These fibers or filaments may be used to produce a single layer or sublayer having different colors or a varied color scheme. For example, concentration of a single pigment may be varied in each hopper connected to the extrusion head, thereby allowing production of a layer having the same general coloration, but with a color scheme including slightly different colors.

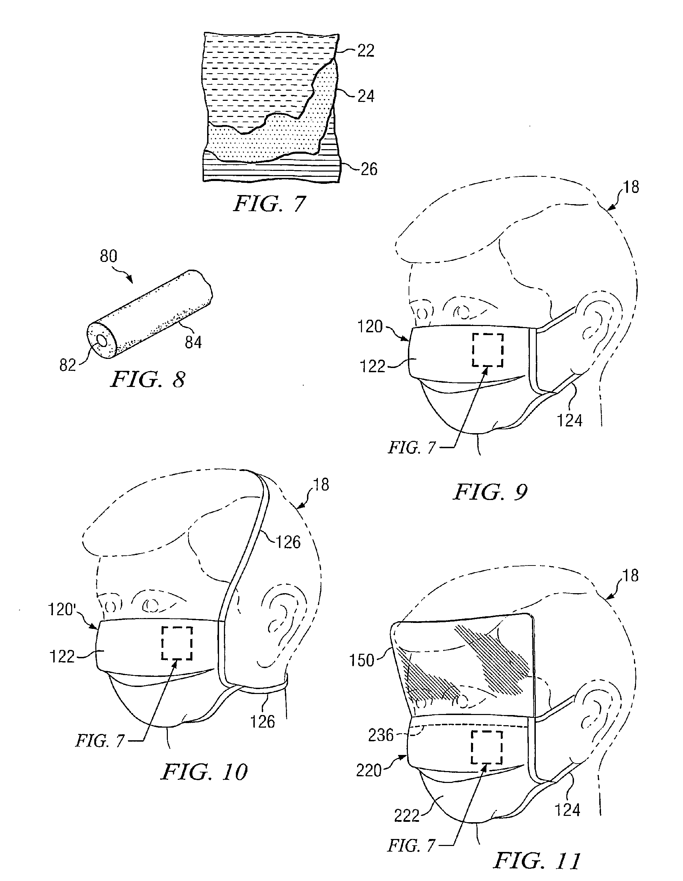

Referring now more particularly to FIGURE 7, for some applications, layer 22 may be formed from spunbonded material. Layer 24 may be formed from meltblown material. Layer 26 may be formed from spunbonded material. Selected color pigments and concentrations may be added to the fibers and/or filaments associated with each layer 22, 24 and 26 to produce a desired color scheme visible from at least one side or surface of respirator 20.

For other applications, respirator 20 may be formed in part with at least one layer of nonwoven material having two or more sublayers. Each sublayer of nonwoven material may be formed from the same type of nonwoven material such as spunbonded or meltblown. Each sublayer of the nonwoven material may have a different color or color scheme. Alternatively, each sublayer may be formed from a different type of nonwoven material and also with a different color scheme formed on each sublayer. In addition to spunbonded and meltblown techniques, nonwoven materials of the present invention may be produced by any known technique for use in generating nonwoven materials. These techniques may include dry laid techniques. For example a separate card may be used for each layer. The fibers or filaments provided on each card may be substantially one color or may vary in color or concentration of pigment. Wet laid techniques may also be used. For example pigment may be added to the slurry used to form each layer or sublayer. The slurry for each layer or sublayer may have substantially the same pigment or concentration throughout, or pigment color or concentration may be varied in different areas of the slurry to produce variation in the layer formed. Nonwoven materials may also be produced using hydroentanglement techniques. For example, pigments may be added to a water jet to produce different color schemes. Other nonwoven material production techniques including, but not limited to, extruding perforated thin plastic films and nonperforated thin plastic films, may also be used.

Color or color schemes in any production technique may be varied by selectively blending or adding pigmented and non-pigmented fibers or filaments. Overall color or color scheme of the completed nonwoven material layer or sublayer may also be affected by concentration of pigments, fiber or filament density, fiber or filament diameter, coverage and spacing, inter alia. More than one of these characteristics may be varied between layers or sublayers or within a given layer or sublayer.

FIGURE 4 shows one example of shoe covering 50 which may be formed from nonwoven materials having a color scheme incorporating teachings of the present invention.

FIGURE 5 shows one example of head covering 60 which may be formed from nonwoven materials having a color scheme incorporating teachings of the present invention.

FIGURE 6 shows one example of a jacket or coat 70 which may be formed from nonwoven materials incorporating teachings of the present invention. Layers of nonwoven material as shown in FIGURE 7 may be used to form shoe covering 50, head covering 60, coat 70, and the outer cover of the masks shown in FIGURES 9-11.

FIGURE 8 is a schematic drawing showing one example of a fiber or filament which may be satisfactorily used to form nonwoven materials and fabrics in accordance with teachings of the present invention. For the embodiment shown in FIGURE 8, bicomponent fiber 80 preferably includes core 82 disposed within hollow sheath 84. Pigments used to form an associated color scheme are preferably added to sheath 84 with only a relatively small percent (less than ten percent) of the pigments added to core 82. Various techniques associated with forming bicomponent fibers and filaments may satisfactorily be used. Typically, one or more hoppers (not expressly shown) may be used to add desired concentration of pigments to sheets 84 during an associated extrusion process which results in only a limited number of pigments being added to core 82. Core 82, in some embodiments may contain no pigment; all pigment may be contained in the sheath 84.

FIGURE 9 is a schematic drawing showing face mask 120 disposed on the face of wearer 18. Face mask 120 may be formed from a wide variety of nonwoven materials including, but not limited to, spunbonded and meltblown materials. Face mask 120 may include mask body 122 formed at least in part from nonwoven materials and ear loops 124. The dimensions and configuration of face mask body 122 may be selected to conform with the face of wearer 18. Ear loops 124 may be formed from various types of elastomeric and/or resilient nonwoven material to provide desired fit and seal with the face of wearer 18.

For the embodiment shown in FIGURE 9, face mask 120 may be formed from at least three layers of nonwoven material. For example, as shown in FIGURES 3 and 7, layer 22 may be formed from spunbonded material. Layer 26 may be formed from spunbonded material. Selected color pigments have preferably been added to the fibers and/or filaments used to form each layer of nonwoven material 22, 24 and 26 to produce a desired color scheme visible from at least one side of face mask 120. For example, appropriate pigments may be added to the fibers used to form layer 22 to produce a color scheme based on robin's egg blue. Appropriate pigments may be added to the fibers used to form layer 24 to produce a color scheme corresponding with wisteria violet. In a similar manner, pigments may be added to layer 26 to produce a color scheme based on seafoam green.

### EXAMPLE

A face mask product having an outer fabric made of SMS as described below has been produced.

| Nonwoven material | % Pigment by weight | Nominal Basis Weight |
|---|---|---|
| Spunbond | 2.4% seagreen | 10 grams/square meter |
| Meltblown | 2% wisteria | 5 grams/square meter |
| Spunbond | 3% robin's egg blue | 10 grams/square meter |

For other applications, face mask 120 may be formed in part from at least one layer of nonwoven material having two or more sublayers. For example, face mask body 122 may be formed from a layer of nonwoven material (not expressly shown) having a first sublayer, second sublayer and a third sublayer. Each sublayer of nonwoven material may be formed from the same type of nonwoven material such as spunbonded or meltblown. Each sublayer of the nonwoven material may have a different color or color scheme such as previously described with respect to layers 22, 24 and 26. Teachings of the present invention may be satisfactorily used to form desired color schemes on multiple layers of different types of nonwoven material or may be used to form desired color schemes on multiple layers of the same type of nonwoven material. Also, the present invention may be used to form desired color schemes on sublayers of the same type of nonwoven material which may then be combined to form a single layer or web of nonwoven material. Finally, the present invention may be used to form desired color schemes including more than one color in the same layer or sublayer.

For some applications, face mask 120 may be formed with an outer layer, a middle filtration layer and an interfacing layer. Each layer may be formed from multiple layers of nonwoven material (not expressly shown). For example, face mask 120 may be formed with an outer layer (not expressly shown) formed from sublayers of spunbonded, meltblown, meltblown and spunbonded material. The outer most layer may also be formed from spunbonded, spunbonded meltblown and spunbonded sublayers or spunbonded, spunbonded and meltblown sublayers. The desired color scheme to indicate performance characteristics of the resulting face mask 120 may be provided in one or more of the sublayers. The middle layer (not expressly shown) may have various sublayers or subcomponents such as meltblown and spunbonded. One or more sublayers of meltblown material may be added to provide desired filtration characteristics and one or more spunbonded layers added to provide a carrier for the meltblown material.

In addition to spunbonded and meltblown techniques, nonwoven materials of the present invention may be produced by any known technique for use in generating nonwoven materials. These techniques may include dry laid techniques. For example, a separate card may be used for each layer. The fibers or filaments provided on each card may be substantially one color or may vary in color or concentration of pigment. Wet laid techniques may also be used. For example, pigment may be added to the slurry used to form each layer or sublayer. The slurry for each layer or sublayer may have substantially the same pigment or concentration throughout, or pigment color of concentration may be varied in different areas of the slurry to produce variation in the layer formed. Nonwoven materials may also be produced using hydroentanglement techniques. For example, pigments may be added to a water jet to produce different color schemes. Other nonwoven material production techniques may also be used.

Color or color scheme in any production technique may be varied by selectively blending or adding pigmented and non-pigmented fibers or filaments. Overall color or color scheme of the completed nonwoven material layer or sublayer may also be affected by concentration of pigments, fiber or filament density, fiber or filament diameter, coverage and spacing, inter alia. More than one of these characteristics may be varied between layers or sublayers or within a given layer or sublayer.

FIGURE 10 is a schematic drawing showing face mask 120' disposed on the face of wearer 18. Face mask 120' may include previously described face mask body 122 along with surgical ties 126. Teachings of the present invention may also be used to provide a desired color scheme with respect to nonwoven materials used to form surgical ties 126. For example, the color scheme associated with surgical ties 126 may correspond with the color scheme of face mask body 122. Alternatively, surgical ties 126 may have a different color scheme to indicate specific functions or characteristics of the associated face mask120'. For some applications, surgical ties 126 may have a unique or specific color scheme while face mask body 122 includes a conventional or standard color scheme.

FIGURE 11 is a schematic drawing showing face mask 220 with visor 150 attached to face mask body 222.

For some applications a region or strip of relatively dark coloration may be formed on face mask body 222 adjacent to visor 150. Dotted line 236 shows one example of a relatively dark region which may be formed on face mask body 222 to reduce glare.

For example, face mask body 222 may be formed with a layer or a sublayer of nonwoven material having a darkened region disposed adjacent to visor 150. Alternatively, bindings (not expressly shown) disposed on the edges of face mask body 222 may be formed from nonwoven materials having a dark or sometimes generally black coloration to reduce glare. The present invention allows adding desired pigmentation to either face mask body 222, ear loops 124 and/or bindings associated with face mask 220 to substantially reduce or eliminate any glare associated with wearing visor 150.

Color schemes may be formed on face mask body 222 bindings and ear loops 124 and/or associated bindings to provide optimum visual performance from wearing face mask 220 based on the intended environment. For example, the present invention allows developing color schemes which may be particularly beneficial for use in laser surgery, or surgery associated with unique lighting environments to better illuminate the surgical field.

FIGURE 7 is a schematic drawing showing layers of nonwoven material 22,24 and 26 having different color schemes formed on each layer in accordance with teachings of the present invention. For some applications each layer 22, 24 and 26 may be formed from the same type of spunbonded material, meltblown material or thin plastic film material. For other applications as represented by face masks 120, 120' and 220, layer 22 may be formed from spunbonded material. Layer 24 may be formed from meltblown material. Layer 26 may be formed from spunbonded material. Alternatively, layers 22, 24 and 26 may represent sublayers of a web or layer of nonwoven material. One or the technical benefits of the present invention includes the ability to form the same type of nonwoven material with different color schemes or to form different types of nonwoven material with different color schemes.

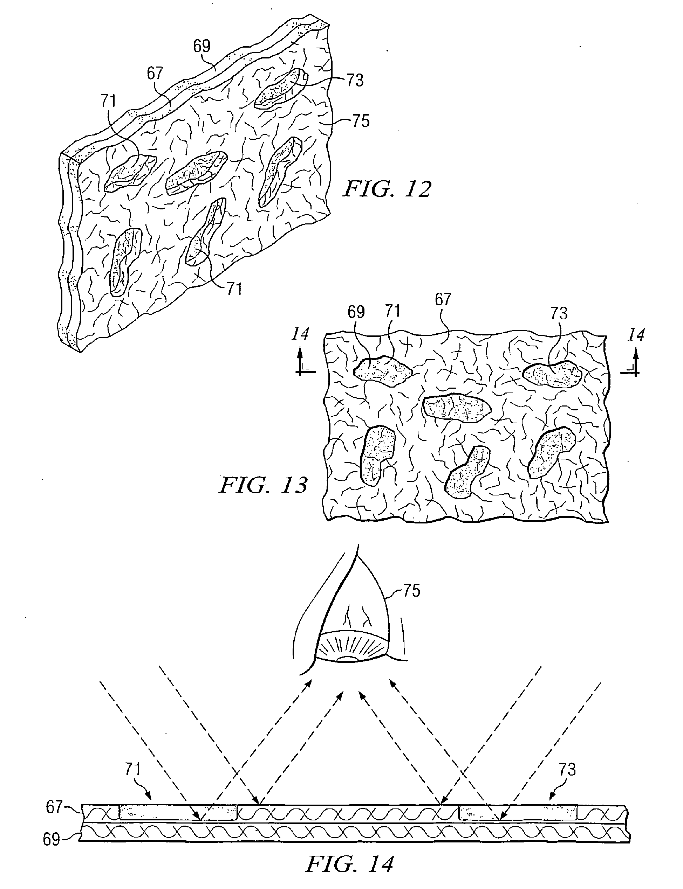

FIGURES 12-14 illustrate a fabric having two layers (or sublayers) 67 and 69 of differentially colored adjacent nonwoven materials. In the embodiment of FIGURES 12-14, at least layer 67, which may be the outer layer of a product made from the fabric, is made by a process in which layer 67 is formed with at least one zone, such as zones 71 and 73, which are more translucent than other portions 75 of layer 67. These more translucent zones may be formed because of the lack of uniformity in the production of the nonwoven material, in particularly in the production of spunbond polypropylene which is formed by randomly deposited entangled filaments. As best illustrated in FIGURE 13, because of the translucence of zones 71 and 73, the color from layer 69 may be more visible through zones 71 and 73 in layer 67. As stated previously, layer 67 may be the outer layer of the nonwoven product constructed from this fabric. Thus, as is best illustrated in FIGURE 14, the human eye 75 will see light reflected from layer 69 through zones 71 and 73, as well as light reflected from the top portion of layer 67. Since layers 67 and 69 are of different colors, the viewer will perceive a multi-colored fabric. In addition, while FIGURE 14 only shows more translucent areas in one layer of the two layer fabric, it is preferred that both layers include zones that are more translucent so that two colors can be observed from either side of the fabric.

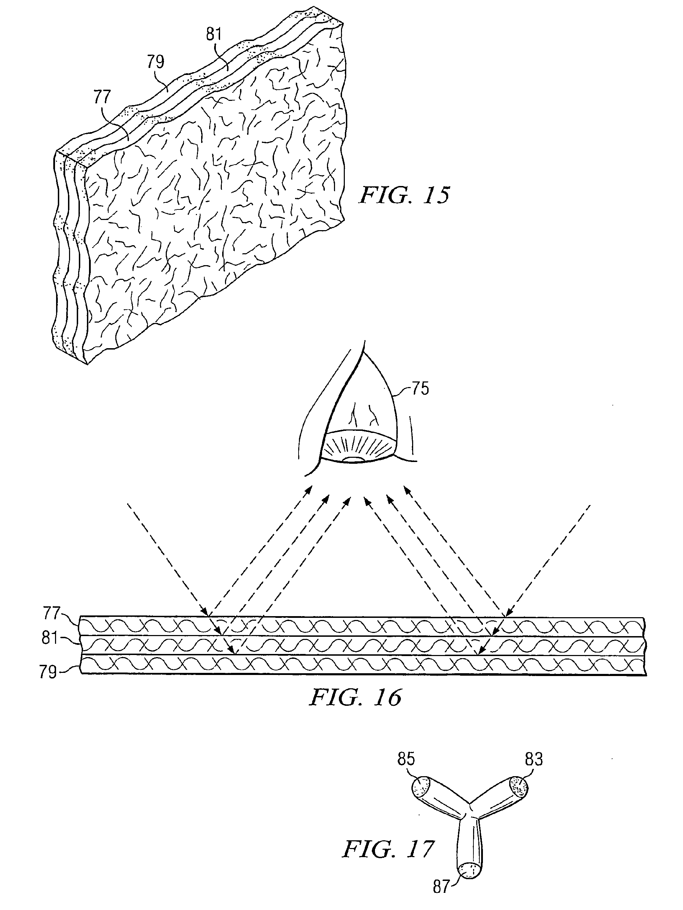

FIGURES 15 and 16 illustrate fabric made of three layers, namely, an outer layer 77, an inner layer 79, with a middle layer 81 sandwiched therebetween. The fabric illustrated in FIGURE 15 may be made by the SMS process shown in FIGURE 26. Each layer 77, 79 and 81 may be made of different colors or different shades of the same color. For one embodiment, the outer layer 77 and the inner layer 79 may be made of substantially the same color with the middle layer 81 being made of a different color. In the embodiment of FIGURES 15 and 16, each layer is made from a more uniform process so that there are few, if any, very thin areas, although at least outer layer 77 and middle layer 81 must be somewhat translucent so that, as illustrated in FIGURE 16, the color from the inner layer 79 must pass through middle layer 81 and outer layer 77 and the colors from middle layer 81 must pass through outer layer 77 so that the human eye will perceive the three layer fabric as a three color fabric. It is also preferred that inner layer 79 be translucent so that three colors may be observed from either side of the fabric.

Nonwoven materials with basis weight less than 2 ounces/square yard (68 grams/square meter) for each layer or sublayer generally allow cooperation between respective colors (different colors or different shades of the same color) of each layer or sublayer in accordance with teachings of the present invention.

Nonwoven materials with typically between 1% and 5% color pigment by basis weight (grams/square meter) generally allow cooperation between respective colors (different colors or different shades of the same color) of each layer or sublayer in accordance with teachings of the present invention.

Nonwoven materials with large diameter fibers or filaments are generally more translucent and allow cooperation between respective colors (different colors or different shades of the same color) of each layer or sublayer in accordance with teachings of the present invention.

Nonwoven materials with basis weights of 2 or 3 ounces or more per square yard (68 or 102 grams or more per square meter) are generally opaque and do not allow cooperation between respective colors (different colors or different shades of the same color) of each layer or sublayer.

FIGURE 17 illustrates a trilobal filament having differential colors or different shades of the same color at the tips of lobes 83, 85 and 87. The use of trilobal filaments may be useful in carrying out the teachings of the invention.

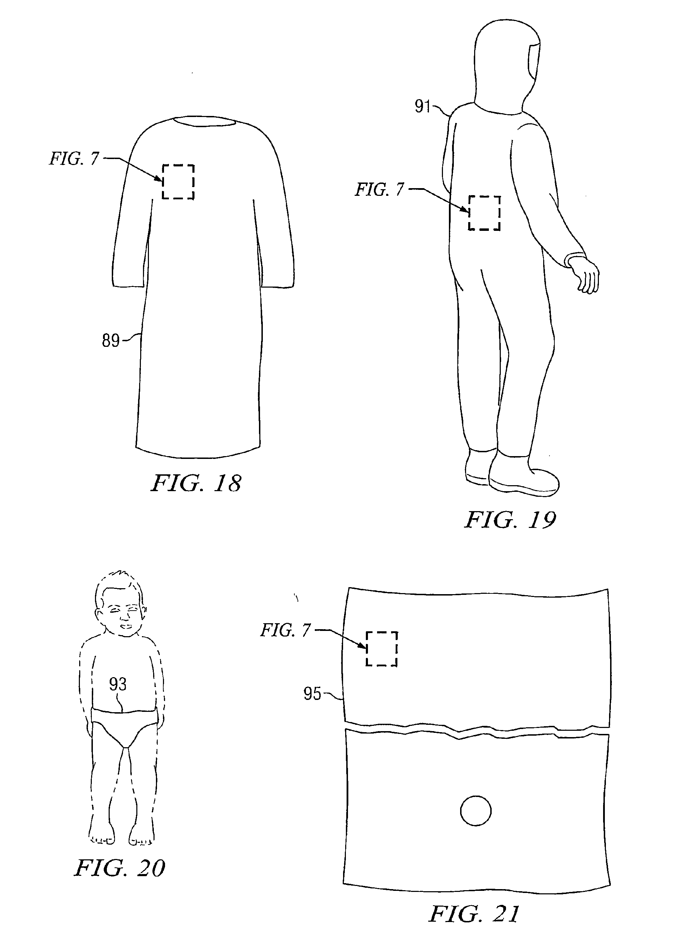

FIGURE 18 shows one example of a gown 89 which may be formed from nonwoven materials having a color scheme incorporating the teachings of the present invention.

FIGURE 19 shows one example of a biological hazard suit 91 which may be formed from nonwoven materials having a color scheme incorporating the teachings of the present invention.

FIGURE 20 shows an infant wearing a diaper 93 which may be formed from nonwoven materials having a color scheme incorporating the teachings of the present invention.

FIGURE 21 shows a surgical drape 95 which may be formed from nonwoven materials having a color scheme incorporating the teachings of the present invention.

FIGURE 22 shows a feminine hygiene product such as a sanitary napkin 97 which may be formed from nonwoven materials having a color scheme incorporating the teachings of the present invention.

FIGURE 23 shows a wipe 99 which may be formed from nonwoven materials having a color scheme incorporating the teachings of the present invention.

FIGURE 24 shows a healthcare worker 101 where hat 60, mask 120, gown 89, and shoe covers 50 which may be formed from nonwoven materials having a color scheme incorporating the teachings of the present invention. Preferably each item which healthcare worker 101 is wearing incorporates the same color scheme so as to show that the healthcare worker is fully protected for a specific environment. For example, the color scheme could be blue-green-purple color scheme to indicate that the healthcare worker is protected from bodily fluids while a yellow-orange-green color scheme could indicate that the healthcare worker is protected from a certain pathogen hazard.

### Industrial Applicability

The way in which the invention is capable of being exploited and the way in which it can be made and used will be apparent from the foregoing.

## Claims

1. A nonwoven product formed with at least two layers of nonwoven material comprising:
the nonwoven material having a color scheme formed by differentially pigmenting associated fibers or filaments;
at least one of the layers of nonwoven material having a color scheme different from the color scheme of one other layer of nonwoven material; and
a color scheme being visible from at least a portion of the product and having a random appearance and being different from the respective color schemes of each layer.

2. The product of claim 1, further comprising the color scheme formed by varying a nonwoven material characteristic selected from the group consisting of:
pigment color, pigment concentration, fiber or filament density, fiber or filament diameter, coverage, spacing and
any combinations thereof.

3. The nonwoven product of claim 1, further comprising:
at least one of the layers of nonwoven material formed from bicomponent fibers defined in part by a core disposed within a hollow sheath;
color pigments used to form the desired color scheme disposed within the sheath and the core; and
the amount of color pigment disposed within the sheath substantially larger than the amount of color pigments disposed within the core.

4. The nonwoven product of claim 3, further comprising substantially no pigment disposed in the core.

5. The nonwoven product of claim 1 , further comprising the color scheme of a first layer, second layer and third layer cooperating with each other to create an appearance of depth.

6. The nonwoven product of claim 1 , further comprising a medical respirator.

7. The nonwoven product of claim 1 , further comprising an industrial respirator.

8. The nonwoven product of claim 1 , further comprising pigments added to the nonwoven material to provide the desired color scheme for each layer.

9. The nonwoven product of claim 1, selected from the group consisting of facial protection masks, medical and dental face masks, medical respirators and industrial respirators.

10. The nonwoven product of claim 1, selected from the group consisting of medical and dental products including caps, gowns, head covers, shoe covers, surgical drapes, sterilization wraps, ice packs, bandages, wound dressings, medical uniforms, and protective garments worn in surgery.

11. The nonwoven product of claim 1, selected from the group consisting of agricultural products including crop covers, sacks, ground covers, weed barriers and seed strips; industrial products including equipment covers, filters, uniforms, wrapping materials, vehicle covers, labels, tags, packaging, envelopes, vehicle headliners, wipes for polishing, cleaning and or disinfecting, and padding; home building products including insulation, house wraps, roofing material, wall covering, and floor pads; personal protection equipment including uniforms, hoods, head covers, gowns, shoe covers, vests, chemical suits, biological protection suits, firefighting suits and biohazard suits; and outdoor products including shade coverings, awnings, camouflage materials, and lawn and patio furniture; personal care products including disposable diapers, undergarments, sanitary napkins, tampons, feminine hygiene products and body wipes; and apparel, upholstery backing materials and interlinings.

12. A method for forming the nonwoven product of any one of claims 1 to 11, comprising:
applying varying concentrations of pigments to form a first color scheme in a first material;
depositing the first material on a substrate;
depositing a second material on said first material;
said first and second materials forming a fabric;
constructing said product from said fabric so that said first material forms an inner layer and said second material forms an outer layer;
and the translucence of said outer layer being such that the color scheme has a random appearance and said first color is visible through at least portions of said outer layer.

13. The method of claim 12, wherein at least portions of said second material are deposited on said first layer non-uniformly, thereby forming substantially translucent regions in said second layer.

14. The method of claim 12, wherein said nonwoven product is selected from the group consisting of facial protection masks, medical and dental face masks, medical respirators and industrial respirators.

15. The method of claim 12, wherein said nonwoven product is selected from the group consisting of medical and dental products including caps, gowns, head covers, shoe covers, surgical drapes, sterilization wraps, ice packs, bandages, wound dressings, medical uniforms, and protective garments worn in surgery.

16. The method of claim 12, wherein said nonwoven product is selected from the group consisting of agricultural products including crop covers, sacks, ground covers, weed barriers and seed strips; industrial products including equipment covers, filters, uniforms, wrapping materials, vehicle covers, labels, tags, packaging, envelopes, vehicle headliners, wipes for polishing, cleaning and or disinfecting, and padding; home building products including insulation, house wraps, roofing material, wall covering, and floor pads; personal protection equipment including uniforms, hoods, head covers, gowns, shoe covers, vests, chemical suits, biological protection suits, firefighting suits and biohazard suits; and outdoor products including shade coverings, awnings, camouflage materials, and lawn and patio furniture; personal care products including disposable diapers, undergarments, sanitary napkins, tampons, feminine hygiene products and body wipes; and apparel, upholstery backing materials and interlinings.

## Patentansprüche

1. Vliesprodukt, das aus zumindest zwei Schichten Vliesmaterial gebildet ist, wobei:
das Vliesmaterial ein Farbmuster aufweist, das durch unterschiedlich pigmentierende, verbundene Fasern oder Filamente gebildet ist;
zumindest eine der Schichten des Vliesmaterials ein Farbmuster aufweist, das sich von dem Farbmuster einer anderen Schicht des Vliesmaterials unterscheidet; und
ein Farbmuster aus zumindest einem Teil des Produkts erkennbar ist und ein zufälliges Erscheinungsbild aufweist und sich von den jeweiligen Farbmustern jeder Schicht unterscheidet.

2. Vliesprodukt nach Anspruch 1, wobei weiters das Farbmuster durch Variieren eines Charakteristikum eines vliesmaterials ausgewählt aus der Gruppe bestehend aus Pigmentfarbe, Pigmentkonzentration, Faser- oder Filamentdichte, Faser- oder Filamentdurchmesser, Beschichtung, Beabstandung und jedwede Kombination davon gebildet ist.

3. Vliesprodukt nach Anspruch 1, wobei weiters:
zumindest eine der Schichten vliesmaterial aus zweikomponentenfasern gebildet ist, die teilweise durch einen Kern definiert sind, der in einer hohlen Hülle angeordnet ist;
Farbpigmente verwendet werden, um das gewünschte Farbmuster zu bilden, das in der Hülle und im Kern vorhanden ist; und
die Menge an Farbpigmenten, die in der Hülle vorhanden ist, im Wesentlichen größer als die Menge an Farbpigmenten im Kern ist.

4. Vliesprodukt nach Anspruch 3, wobei weiters im Wesentlichen kein Pigment im Kern vorhanden ist.

5. Vliesprodukt nach Anspruch 1, wobei weiters das Farbmuster einer ersten Schicht, einer zweiten Schicht und einer dritten Schicht zusammenwirken, um ein tiefes Erscheinungsbild zu schaffen.

6. Vliesprodukt nach Anspruch 1, weiters mit einem medizinischen Respirator.

7. Vliesprodukt nach Anspruch 1, weiters mit einem industriellen Respirator.

8. Vliesprodukt nach Anspruch 1, wobei dem Vliesmaterial weiters Pigmente zugegeben werden, um das gewünschte Farbmuster für jede Schicht bereitzustellen.

9. Vliesprodukt nach Anspruch 1, ausgewählt aus der Gruppe bestehend aus Gesichtsschutzmasken, medizinischen und zahnmedizinischen Gesichtsmasken, medizinischen Respiratoren und industriellen Respiratoren.

10. Vliesprodukt nach Anspruch 1, ausgewählt aus der Gruppe bestehend aus medizinischen und zahnmedizinischen Produkten, einschließlich Mützen, Kittel, Kopfabdeckungen, Schuhabdeckungen, chirurgische Abdecktücher, Sterilisationsumhänge, Eispackungen, Bandagen, Wundverbandmaterial, medizinische Uniformen und Schutzkleidung, die bei einer Operation getragen wird.

11. Vliesprodukt nach Anspruch 1, ausgewählt aus der Gruppe bestehend aus landwirtschaftlichen Produkten, einschließlich Ernteabdeckungen, Säcke, Bodenabdeckungen, Unkrautsperrmittel und Saatstreifen; industriellen Produkten, einschließlich Gerätschaftabdeckungen, Filter, Uniformen, Umhüllungsmaterialien, Fahrzeugabdeckungen, Schilder, Anhänger, Verpackungen, Umhüllungen, Fahrzeugdachverkleidungen, Tücher zum Polieren, Reinigen und/oder Desinfizieren, und Füllmaterial; Heimwerkerprodukten, einschließlich Wärmedämmung, Hausumhüllungen, Dachmaterialien, Wandabdeckung und Bodenbeläge; persönlichen Schutzausstattungen, einschließlich Uniformen, Hauben, Kopfabdeckung, Kittel, Schuhabdeckungen, Westen, chemische Anzüge, biologische Schutzanzüge, Brandschutzanzüge und Biogefährdungs-Schutzanzüge; und Freiluftprodukten, einschließlich Lichtschutzabdeckungen, Wetterschutzabdeckungen, Sichtschutzmaterialien und Rasen- und Terrassenmöbel; persönlichen Pflegeprodukten, einschließlich Einwegwindeln, Unterkleider, Damenbinden, Tampons, weibliche Hygieneprodukte und Körpertücher; und Bekleidungs-, Polsterungsgrundmaterialien und Futterstoffen.

12. Verfahren zum Bilden des Vliesprodukts nach einem der Ansprüche 1 bis 11, umfassend:
Anwenden von variierenden Pigmentkonzentrationen, um ein erstes Farbmuster in einem ersten Material zu bilden;
Legen des ersten Materials auf ein Substrat;
Legen eines zweiten Materials auf das erste Material;
wobei das erste und zweite Material ein Gewebe bilden;
Herstellen des Produktes aus dem Gewebe, so dass das erste Material eine Innenschicht und das zweite Material eine Außenschicht bildet; und
wobei die Lichtdurchlässigkeit der Außenschicht so ist, dass das Farbmuster ein zufälliges Erscheinungsbild aufweist, und dass die erste Farbe durch zumindest Teile der Außenschicht erkennbar ist.

13. Verfahren nach Anspruch 12, wobei zumindest Teile des zweiten Materials nicht einheitlich auf die erste Schicht gelegt sind, wodurch im Wesentlichen lichtdurchlässige Bereiche in der zweiten Schicht gebildet werden.

14. Verfahren nach Anspruch 12, wobei das Vliesprodukt ausgewählt aus der Gruppe bestehend aus Gesichtsschutzmasken, medizinischen und zahnmedizinischen Gesichtsmasken, medizinischen Respiratoren und industriellen Respiratoren.

15. Verfahren nach Anspruch 12, wobei das Vliesprodukt ausgewählt ist aus der Gruppe bestehend aus medizinischen und zahnmedizinischen Produkten, einschließlich Mützen, Kittel, Kopfabdeckungen, Schuhabdeckungen, chirurgische Abdecktücher, Sterilisationsumhänge, Eispackungen, Bandagen, Wundverbandmaterial, medizinische Uniformen und Schutzkleidung, die bei einer Operation getragen wird.

16. Verfahren nach Anspruch 12, wobei das Vliesprodukt ausgewählt ist aus der Gruppe bestehend aus landwirtschaftlichen Produkten, einschließlich Ernteabdeckungen, Säcke, Bodenabdeckungen, Unkrautsperrmittel und Saatstreifen; industriellen Produkten, einschließlich Gerätschaftabdeckungen, Filter, Uniformen, Umhüllungsmaterialien, Fahrzeugabdeckungen, Schilder, Anhänger, Verpackungen, Umhüllungen, Fahrzeugdachverkleidungen, Tücher zum Polieren, Reinigen und/oder Desinfizieren, und Füllmaterial; Heimwerkerprodukten, einschließlich Wärmedämmung, Hausumhüllungen, Dachmaterialien, Wandabdeckung und Bodenbeläge; persönlichen Schutzausstattungen, einschließlich Uniformen, Hauben, Kopfabdeckung, Kittel, Schuhabdeckungen, Westen, chemische Anzüge, biologische Schutzanzüge, Brandschutzanzüge und Biogefährdungs-Schutzanzüge; und Freiluftprodukten, einschließlich Lichtschutzabdeckungen, Wetterschutzabdeckungen, Sichtschutzmaterialien und Rasen- und Terrassenmöbel; persönlichen Pflegeprodukten, einschließlich Einwegwindeln, Unterkleider, Damenbinden, Tampons, weibliche Hygieneprodukte und Körpertücher; und Bekleidungs-, Polsterungsgrundmaterialien und Futterstoffen.

## Revendications

1. Produit non tissé formé avec au moins deux couches de matériau non tissé comprenant :
le matériau non tissé présentant un agencement de couleurs formé en pigmentant différemment des fibres ou filaments associé(e)s ;
au moins une des couches de matériau non tissé présentant un agencement de couleurs différent de l' agencement de couleurs d'une autre couche de matériau non tissé ; et
un agencement de couleurs étant visible depuis au moins une partie du produit et présentant une apparence aléatoire et étant différent des agencements de couleurs respectifs de chaque couche.

2. Produit selon la revendication 1, comprenant en outre l'agencement de couleurs formé en modifiant une caractéristique de matériau non tissé sélectionnée à partir du groupe constitué par : la couleur du pigment, la concentration du pigment, la densité de la fibre ou du filament, le diamètre de la fibre ou du filament, le champ couvert, l'espacement et n'importe quelle combinaison de celles-ci.

3. Produit non tissé selon la revendication 1, comprenant en outre :
au moins une des couches de matériau non tissé formée à partir de fibres à deux composants définies en partie par une âme disposée à l'intérieur d'une gaine creuse ;
des pigments colorés utilisés pour former l'agencement de couleurs souhaité disposé à l'intérieur de la gaine et de l'âme ; et
la quantité de pigment coloré disposé à l'intérieur de la gaine sensiblement supérieure à la quantité de pigments colorés disposés à l'intérieur de l'âme.

4. Produit non tissé selon la revendication 3, ne comprenant en outre sensiblement aucun pigment disposé dans l'âme.

5. Produit non tissé selon la revendication 1, comprenant en outre l'agencement de couleurs d'une première couche, d'une deuxième couche et d'une troisième couche coopérant les unes avec les autres pour créer une apparence de profondeur.

6. Produit non tissé selon la revendication 1, comprenant en outre un respirateur médical.

7. Produit non tissé selon la revendication 1, comprenant en outre un respirateur industriel.

8. Produit non tissé selon la revendication 1, comprenant en outre des pigments ajoutés au matériau non tissé pour fournir l'agencement de couleurs souhaité pour chaque couche.

9. Produit non tissé selon la revendication 1, sélectionné à partir du groupe constitué par les masques de protection faciale, les masques faciaux médicaux et dentaires, les respirateurs médicaux et les respirateurs industriels.

10. Produit non tissé selon la revendication 1, sélectionné à partir des groupes constitués par les produits médicaux et dentaires incluant les coiffes, les blouses, les calottes, les couvre-chaussures, les draps chirurgicaux, les emballages pour stérilisation, les vessies de glace, les bandages, les pansements, les uniformes médicaux, et les vêtements protecteurs portés en chirurgie.

11. Produit non tissé selon la revendication 1, sélectionné à partir du groupe constitué par les produits agricoles incluant les couvre-récolte, les sacs, les couvre-sol, les dispositifs anti-mauvaises herbes et les rubans de semence ; les produits industriels incluant les couvertures pour équipement, les filtres, les uniformes, les matériaux d'enveloppement, les couvertures pour véhicule, les labels, les étiquettes, les emballages, les enveloppes, les garnitures de toit de véhicule, les lingettes polissantes, nettoyantes et/ou désinfectantes, et les tampons ; les produits de construction individuelle incluant l'isolation, les membranes pare-air, le matériau de couverture, le revêtement mural, et les tapis protecteurs ; l'équipement de protection personnel incluant les uniformes, les capuches, les calottes, les blouses, les couvre-chaussures, les vestes, les combinaisons de protection chimique, les combinaisons de protection biologique, les combinaisons de protection contre le feu et les combinaisons de protection contre les risques biologiques ; et les produits de plein air incluant les couvre-store, les marquises, les matériaux de camouflage, et les fourniture pour gazon et terrasse ; les produits de soins personnels incluant les couches jetables, les sous-vêtements, les serviettes hygiéniques, les tampons, les produits pour l'hygiène féminine et les lingettes corporelles ; et les vêtements, les matériaux de garniture et triplures.

12. Procédé de formation du produit non tissé selon l'une quelconque des revendications 1 à 11, comprenant les étapes consistant à :
appliquer diverses concentrations de pigments pour former un premier agencement de couleurs dans un premier matériau ;
déposer le premier matériau sur un substrat ;
déposer un second matériau sur ledit premier matériau ;
lesdits premier et second matériaux formant un tissu ;
construire ledit produit à partir dudit tissu de sorte que ledit premier matériau forme une couche interne et ledit second matériau forme une couche externe;
et la transparence de ladite couche externe étant telle que l'agencement de couleurs présente une apparence aléatoire et que ladite première couleur est visible à travers au moins des parties de ladite couche externe.

13. Procédé selon la revendication 12, dans lequel au moins des parties dudit second matériau sont déposées sur ladite première couche de manière non uniforme, formant de ce fait des zones sensiblement transparentes dans ladite seconde couche.

14. Procédé selon la revendication 12, dans lequel ledit produit non tissé est sélectionné à partir du groupe constitué par les masques de protection faciaux, les masques faciaux médicaux et dentaires, les respirateurs médicaux et les respirateurs industriels.

15. Procédé selon la revendication 12, dans lequel ledit produit non tissé est sélectionné à partir du groupe constitué par les produits médicaux et dentaires incluant les coiffes, les blouses, les calottes, les couvre-chaussures, les draps chirurgicaux, les emballages pour stérilisation, les vessies de glace, les bandages, les pansements, les uniformes médicaux, et les vêtements protecteurs portés en chirurgie.

16. Procédé selon la revendication 12, dans lequel ledit produit non tissé est sélectionné à partir du groupe constitué par les produits agricoles comportant les couvre-récolte, les sacs, les couvre-sol, les dispositifs anti-mauvaises herbes et les rubans de semence ; les produits industriels incluant les couvertures pour équipement, les filtres, les uniformes, les matériaux d'enveloppement, les couvertures pour véhicule, les labels, les étiquettes, les emballages, les enveloppes, les garnitures de toit de véhicule, les lingettes polissantes, nettoyantes et/ou désinfectantes, et les tampons ; les produits de construction individuelle incluant l'isolation, les membranes pare-air, le matériau de couverture, le revêtement mural, et les tapis protecteurs ; l'équipement de protection personnel incluant les uniformes, les capuches, les calottes, les blouses, les couvre-chaussures, les vestes, les combinaisons de protection chimique, les combinaisons de protection biologique, les combinaisons de protection contre le feu et les combinaisons de protection contre les risques biologiques ; et les produits de plein air incluant les couvre-store, les marquises, les matériaux de camouflage, et les fourniture pour gazon et terrasse ; les produits d'hygiène personnelle incluant les couches jetables, les sous-vêtements, les serviettes hygiéniques, les tampons, les produits pour l'hygiène féminine et les lingettes corporelles ; et les vêtements, les matériaux de garniture et triplures.
